(19) Europäisches Patentamt
European Patent Office
Office européen des brevets

(11) **EP 4 487 760 A1**

(12) **EUROPEAN PATENT APPLICATION**
published in accordance with Art. 153(4) EPC

(43) Date of publication:
**08.01.2025 Bulletin 2025/02**

(21) Application number: **23759778.6**

(22) Date of filing: **14.02.2023**

(51) International Patent Classification (IPC):
**A61B 5/00** (2006.01) **A61B 5/055** (2006.01)

(52) Cooperative Patent Classification (CPC):
**A61B 5/442; A61B 5/0265; A61B 5/0285; G16H 10/20; G16H 30/40;** A61B 5/055

(86) International application number:
**PCT/JP2023/004952**

(87) International publication number:
**WO 2023/162776 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.02.2022 JP 2022030316**

(71) Applicant: **Shiseido Company, Ltd.**
**Chuo-ku**
**Tokyo 104-0061 (JP)**

(72) Inventors:
• **TAKAI, Eisuke**
**Tokyo 104-0061 (JP)**
• **TAMADA, Daiki**
**Kofu-shi, Yamanashi 400-8510 (JP)**

(74) Representative: **Ter Meer Steinmeister & Partner**
**Patentanwälte mbB**
**Nymphenburger Straße 4**
**80335 München (DE)**

(54) **EVALUATION METHOD, EVALUATION DEVICE, AND PROGRAM**

(57) The state of blood vessels deep in the face is easily identified. An evaluation method includes acquiring information relating to an evaluation target person, the information including an actual age of the evaluation target person and an answer to a questionnaire about skin of a face of the evaluation target person; and predicting a state of a blood vessel in a deep part of the face of the evaluation target person from the information relating to the evaluation target person.

FIG.3

START

S101
ACQUIRE ACTUAL AGE OF EVALUATION TARGET AND QUESTIONNAIRE ANSWERS ABOUT SKIN OF FACE OF EVALUATION TARGET PERSON

S102
PREDICT STATE OF BLOOD VESSEL IN DEEP PART OF FACE OF EVALUATION TARGET PERSON

S103
PREDICT MEASUREMENT VALUE OF SKIN OF FACE OF EVALUATION TARGET PERSON

S104
PRESENT BEAUTY INFORMATION

END

EP 4 487 760 A1

# Description

Technical Field

The present invention relates to an evaluation method, an evaluation apparatus, and a program.

Background Art

Conventionally, methods for identifying the state of a blood vessel are known. For example, methods such as OCTA (Optical Coherence Tomography Angiography), a blood flowmeter, Photoacoustic Imaging, and FMD (Flow Mediated Dilatation) are known.

Citation List

Patent Document

Patent Document 1: Japanese Unexamined Patent Application Publication No. 2008-188428

Summary of Invention

Technical Problem

Conventionally, however, there has been no method for obtaining information on the state of blood vessels deep in the face in a non-invasive manner.

Accordingly, it is an object of an embodiment of the present invention to easily identify the state of blood vessels deep in the face.

Solution to Problem

An evaluation method according to an embodiment of the present invention includes acquiring information relating to an evaluation target person, the information including an actual age of the evaluation target person and an answer to a questionnaire about skin of a face of the evaluation target person; and predicting a state of a blood vessel in a deep part of the face of the evaluation target person from the information relating to the evaluation target person.

Advantageous Effects of Invention

According to an embodiment of the present invention, the state of blood vessels deep in the face can be easily identified.

Brief Description of Drawings

[FIG. 1] FIG. 1 is an overall configuration diagram according to an embodiment of the present invention.

[FIG. 2] FIG. 2 is a functional block diagram of an evaluation apparatus according to an embodiment of the present invention.

[FIG. 3] FIG. 3 is a flowchart (first example) of an evaluation process according to an embodiment of the present invention.

[FIG. 4] FIG. 4 is a flowchart (second example) of an evaluation process according to an embodiment of the present invention.

[FIG. 5] FIG. 5 is a diagram for explaining the correspondence relationship between "actual age and facial skin questionnaire answers" and "state of blood vessels deep in the face" according to an embodiment of the present invention.

[FIG. 6] FIG. 6 is a diagram for explaining the correspondence relationship between "actual age and facial skin questionnaire answers" and "state of blood vessels deep in the face" according to an embodiment of the present invention.

[FIG. 7] FIG. 7 is a diagram for explaining the correspondence relationship between "actual age and facial skin questionnaire answers" and "state of blood vessels deep in the face" according to an embodiment of the present invention.

[FIG. 8] FIG. 8 is a diagram for explaining the relationship between state of blood vessels and amount of sagging around the eyes according to an embodiment of the present invention.

[FIG. 9] FIG. 9 is a diagram for explaining the relationship between state of blood vessels and amount of sagging around the eyes and actual age according to an embodiment of the present invention.

[FIG. 10] FIG. 10 is a diagram for explaining the relationship between actual age and state of blood vessels according to an embodiment of the present invention.

[FIG. 11] FIG. 11 is a block diagram illustrating an example of a hardware configuration of an evaluation apparatus according to an embodiment of the present invention.

Description of Embodiments

Hereinafter, an embodiment of the present invention will be described by referring to the drawings.

<Explanation of terms>

In the present specification, a "blood vessel in the deep part of the face" is an artery in the face (specifically, the buccal artery).

<Overall configuration>

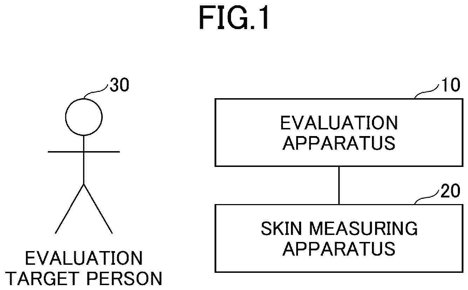

FIG. 1 is an overall configuration diagram according to an embodiment of the present invention. Each of the elements will be described below.

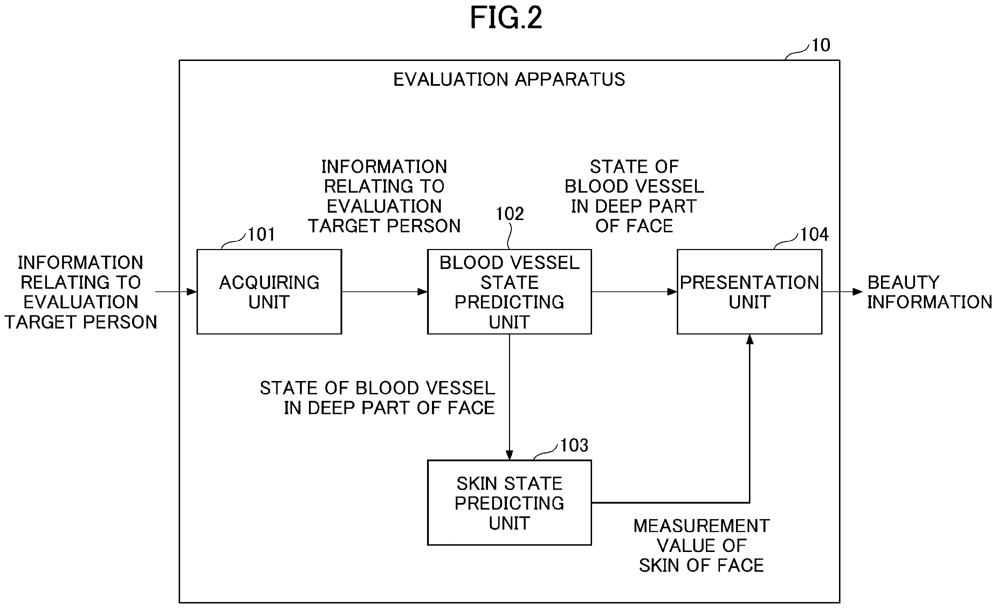

An evaluation apparatus 10 evaluates the state of blood vessels deep in the face of an evaluation target person 30. More specifically, the evaluation apparatus 10 predicts the state of blood vessels deep in the face of the evaluation target person 30 from information relating to the evaluation target person 30 (for example, "actual age and facial skin questionnaire answers" or "facial skin measurement value"). The evaluation apparatus 10 includes one or more computers. In a later stage, the evaluation apparatus 10 will be described in detail by referring to FIG. 2.

A skin measuring apparatus 20 measures the skin of the face of the evaluation target person 30. For example, the measurement value of the skin of the face is the amount of sagging around the eyes.

<Functional block>

FIG. 2 is a functional block diagram of the evaluation apparatus 10 according to an embodiment of the present invention. The evaluation apparatus 10 may include an acquiring unit 101, a blood vessel state predicting unit 102, a skin state predicting unit 103, and a presentation unit 104. The evaluation apparatus 10 can function as the acquiring unit 101, the blood vessel state predicting unit 102, the skin state predicting unit 103, and the presentation unit 104 by executing a program. The blood vessel state predicting unit 102 and the skin state predicting unit 103 are collectively referred to as a predicting unit.

The acquiring unit 101 acquires information relating to the evaluation target person 30.

<Information relating to evaluation target person>

Information relating to the evaluation target person 30 will now be described. Hereinafter, two examples of information relating to the evaluation target person 30 will be described.

<<Actual age and responses to questionnaire on facial skin>>

For example, the information relating to the evaluation target person 30 includes the actual age of the evaluation target person 30 and the response to the facial skin questionnaire of the evaluation target person 30. For example, the questionnaire is a questionnaire about sagging around the eyes.

For example, the questionnaire includes at least one of the following questions:

[Examples of questions and responses of questionnaire]

- Feeling [1] or not feeling [0] "oiliness".

    * Regardless of whether or not you suffer from "oiliness".

- Feeling [1] or not feeling [0] "dullness".

    * Regardless of whether or not you suffer from "dullness".

- Feeling [1] or not feeling [0] "undereye sagging".

    * Regardless of whether or not you suffer from "undereye sagging".

- Feeling [1] or not feeling [0] "rough lips".

    * Regardless of whether or not you suffer from "rough lips".

- Suffering [1] or not suffering [0] "dullness".
- Suffering [1] or not suffering [0] "noticeable spots and freckles".
- Suffering [1] or not suffering [0] "undereye sagging".
- Suffering [1] or not suffering [0] "noticeable eye bag".

<<Measurement value of facial skin>>

For example, information relating to the evaluation target person 30 includes a measurement value of the facial skin. For example, the measurement value of the skin of the face is a measurement value of sagging around the eyes. Specifically, the measurement value of the skin of the face is the amount of sagging around the eyes.

The "amount of sagging around the eyes" is the volume (cc) of a portion (around the eyes) that is more depressed when the evaluation target person 30 is in the vertical position than when the evaluation target person 30 is in the horizontal position. "Around the eyes" refers to a region of the face defined by the upper limit being the lower eyelid and the lower limit being the cheekbone, sandwiched laterally between the nose and sideburns or ears, and one of the two regions exist on each of the sides of the nose. The "horizontal position" refers to a state in which the midline of the face is stationary at a right angle to the direction of gravity. The "vertical position" refers to a state in which the midline of the face is stationary and parallel to the direction of gravity.

The blood vessel state predicting unit 102 predicts a state of blood vessels deep in the face of the evaluation target person 30 from information relating to the evaluation target person 30 acquired by the acquiring unit 101. Specifically, the blood vessel state predicting unit 102 predicts a state of blood vessels deep in the face of the evaluation target person 30 by referring to a predetermined correspondence relationship between "information relating to the evaluation target person 30" and "a state of blood vessels deep in the face" (described later in detail).

For example, the state of the blood vessel in the deep part of the face is the state of the blood vessel calculated from the blood flow velocity in the blood vessel. For example, the state of the blood vessel in the deep part of the face is the state of the blood vessel calculated from the blood flow rate and the radius of the blood vessel.

The skin state predicting unit 103 predicts a measurement value (for example, the amount of sagging around the eyes) of the skin of the face of the evaluation target person 30 from the state of the blood vessel in the deep part of the face of the evaluation target person 30 predicted by the blood vessel state predicting unit 102. Specifically, the skin state predicting unit 103 predicts a measurement value (for example, the amount of sagging around the eyes) of the skin of the face of the evaluation target person 30 by referring to a predetermined correspondence relationship between the "state of the blood vessel in the deep part of the face" and "facial skin measurement value (for example, the amount of sagging around the eyes)".

The presentation unit 104 presents beauty information corresponding to the state of the blood vessel in the deep part of the face of the evaluation target person 30. Specifically, the presentation unit 104 extracts and presents beauty information corresponding to the state of the blood vessel in the deep part of the face of the evaluation target person 30 by referring to a predetermined correspondence relationship between the "state of the blood vessel in the deep part of the face" and "beauty information suitable for the state of the blood vessel". The presentation unit 104 may present beauty information corresponding to the measurement value (for example, the amount of sagging around the eyes) of the skin of the face predicted by the skin state predicting unit 103.

<<Beauty information>>

Here, beauty information will be described. The beauty information is information relating to products and services related to beauty (for example, product name, service name, price, etc.). For example, the beauty information includes information relating to cosmetics for skin care and makeup, information relating to foods and drinks such as supplements, information relating to cosmetic equipment, information relating to beauty gear, information relating to esthetics, and a beauty method to be performed by the evaluation target person 30.

<Method>

Two examples of evaluation processing will be described below.

<First example>

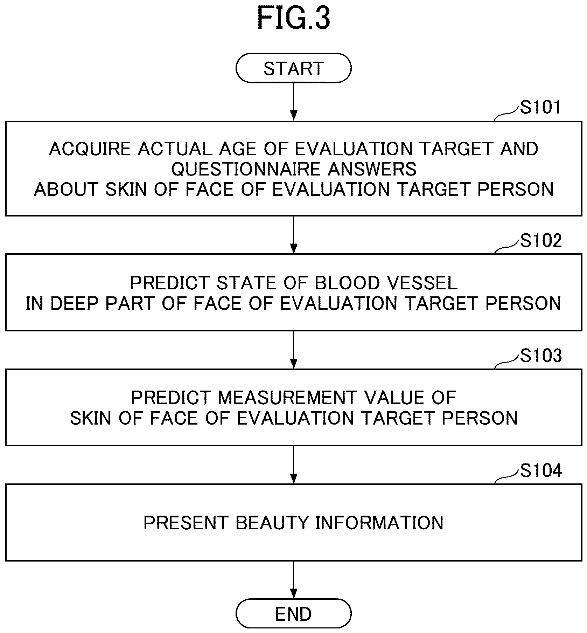

FIG. 3 is an example of evaluation processing (first example) according to an embodiment of the present invention.

In step 101 (S101), the acquiring unit 101 acquires information relating to the evaluation target person 30 including the actual age of the evaluation target person 30 and the answer to the questionnaire on the facial skin of the evaluation target person 30.

In step 102 (S102), the blood vessel state predicting unit 102 predicts the blood vessel state deep in the face of the evaluation target person 30 from the information relating to the evaluation target person 30 acquired in S101.

In step 103 (S103), the skin state predicting unit 103 predicts a measurement value (for example, the amount of sagging around the eyes) of the facial skin of the evaluation target person 30 from the blood vessel state deep in the face of the evaluation target person 30 predicted in S102.

In step 104 (S104), the presentation unit 104 presents beauty information corresponding to the blood vessel state deep in the face of the evaluation target person 30 predicted in S102. The presentation unit 104 may present beauty information corresponding to the measurement value (for example, the amount of sagging around the eyes) of the facial skin predicted in S103.

<Second example>

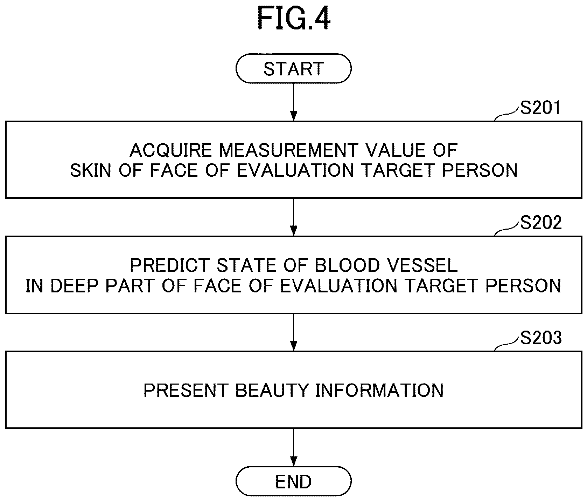

FIG. 4 is an example of evaluation processing (second example) according to an embodiment of the present invention.

In step 201 (S201), the acquiring unit 101 acquires information relating to the evaluation target person 30 including a measurement value (for example, the amount of sagging around the eyes) of the skin of the face of the evaluation target person 30.

In step 202 (S202), the blood vessel state predicting unit 102 predicts the state of blood vessels deep in the face of the evaluation target person 30 from the information relating to the evaluation target person 30 acquired in S201.

In step 203 (S203), the presentation unit 104 presents beauty information according to the state of blood vessels deep in the face of the evaluation target person 30 predicted in S202.

<State of blood vessels in the deep part of the face>

Here, the state of blood vessels (e.g., arteries in the face) in the deep part of the face will be described.

In the present invention, the intensity of MRI (Magnetic Resonance Imaging) is used as an index (health of blood vessels) for quantifying the state of blood vessels in the deep part of the face. Specifically, in all images acquired by MRA-PC (Magnetic Resonance Angiography-Phase Contrast) from a subject, the maximum intensity of brightness is calculated within a range of 10P (pixels) $\times$ 10P (pixels) of the position of blood vessels, and the average value of all maximum intensities is defined as the intensity of blood vessel brightness in the deep part of the face. The measurement parameter is set so that the intensity of blood vessel brightness in the deep part of the face is proportional to the blood flow velocity, and the concept of quantifying the state of blood vessels in the deep part of the face will be described in detail below.

The following formula is derived from fluid dynamics.

$$v = Q/\pi r^2 \text{ ...(formula 1)}$$

Note that:

v: blood flow velocity
Q: blood flow rate
r: radius of blood vessel

It is known that the radius of a blood vessel (e.g., arteries in the face) in the deep part of the face decreases with aging. From the above formula (1), the blood flow velocity is inversely proportional to the square of the radius of a blood vessel. That is, if the intensity of a blood vessel brightness is used as an index for quantifying the state of a blood vessel in the deep part of the face, the health of a blood vessel can be quantified with high sensitivity. For example, if the blood flow velocity is fast, which can be said to be an unhealthy blood vessel, the blood vessel becomes bright, and if the blood flow velocity is slow, which can be said to be a healthy blood vessel, the blood vessel becomes dark. From these facts, it can be seen that the brighter blood vessel (that is, the square of the radius of the blood vessel is small) is the less healthy blood vessel, and the darker blood vessel (that is, the square of the radius of the blood vessel is greater) is the healthier blood vessel.

<Correspondence relationship between "information relating to the evaluation target person" and "state of blood vessels in the deep part of the face">

The correspondence relationship between "information relating to the evaluation target person 30" and "state of blood vessels in the deep part of the face" will now be described. Descriptions will be made for a case where "information relating to the evaluation target person 30" includes "actual age and facial skin questionnaire answers" and a case where "information relating to the evaluation target person 30" includes "a measurement value of the skin of the face".

<<Correspondence relationship between "actual age and facial skin questionnaire answers" and "the state of blood vessels in the deep part of the face">>

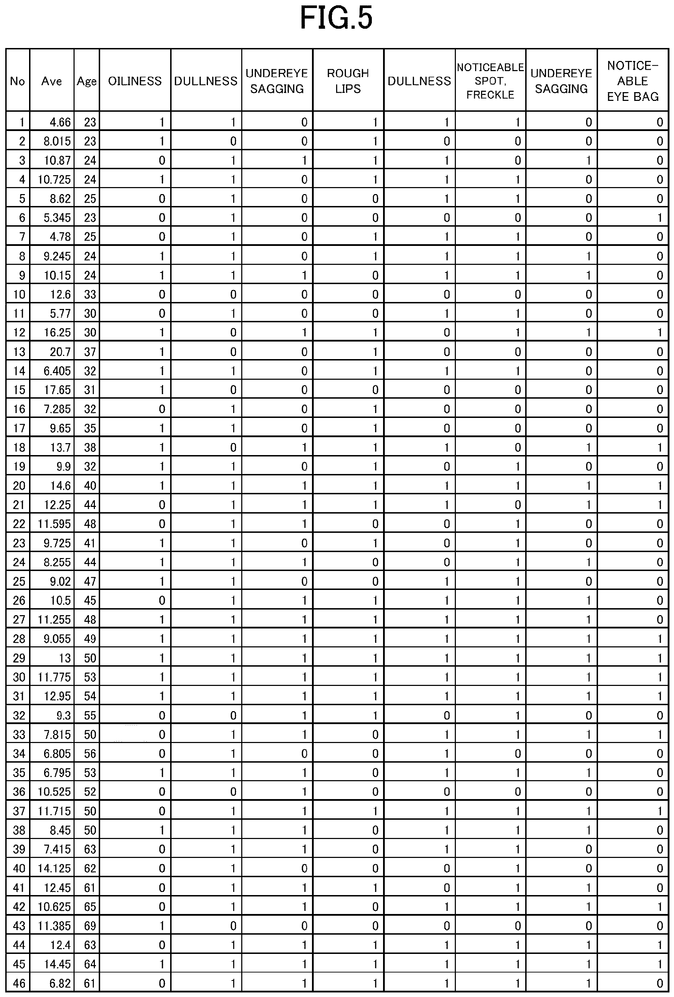

A case where information relating to the evaluation target person 30 includes "actual age and facial skin questionnaire answers" will be described. FIGS. 5 to 7 are diagrams for explaining the correspondence relationship between "actual age and facial skin questionnaire answers" and "state of blood vessels in the deep part of the face" according to an embodiment of the present invention.

When the following questionnaire was implemented for the subjects (46 persons), the answers indicated in FIG. 5 were obtained.

[Example of questions and answers to the questionnaire]

* Feeling [1] or not feeling [0] "oiliness".

    * Regardless of whether or not you suffer from "oiliness".

* Feeling [1] or not feeling [0] "dullness".

    * Regardless of whether or not you suffer from "dullness".

* Feeling [1] or not feeling [0] "undereye sagging".

    * Regardless of whether or not you suffer from "undereye sagging".

* Feeling [1] or not feeling [0] "rough lips".

    * Regardless of whether or not you suffer from "rough lips".

* Suffering [1] or not suffering [0] "dullness".
* Suffering [1] or not suffering [0] "noticeable spots and freckles".
* Suffering [1] or not suffering [0] "undereye sagging".
* Suffering [1] or not suffering [0] "noticeable eye bag".

"No" in FIG. 5 indicates the subject number. "Ave" in FIG. 5 indicates the state of blood vessels (health of blood vessels) deep in the subject's face based on the intensity of brightness of blood vessel obtained from MRI images. "Age" in FIG. 5 indicates the actual age of the subject. In FIG. 5, "oiliness (feeling it, not feeling it)", "dullness (feeling it, not feeling it)", "undereye sagging (feeling it, not feeling it)", "rough lips (feeling it, not feeling it)", "dullness (suffering from it, not suffering from it)", "noticeable spots and freckles (suffering from it, not suffering from it)", "undereye sagging (suffering from it, not suffering from it)", and "noticeable eye bag (suffering from it, not suffering from it)" indicate questions of the questionnaire.

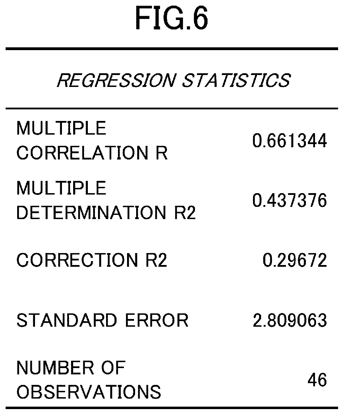

FIG. 6 illustrates regression statistics between "actual age and facial skin questionnaire answers" and "the state of blood vessels in the deep part of the face". As illustrated in FIG. 6, the multiple correlation R (multiple correlation coefficient) is 0.661344, the multiple determination $R^2$ (coefficient of determination) is 0.437376, the correction $R^2$ (DOF adjusted or DOF corrected coefficient of determination) is 0.29672, the standard error is 2.809063, and the number of observations is 46.

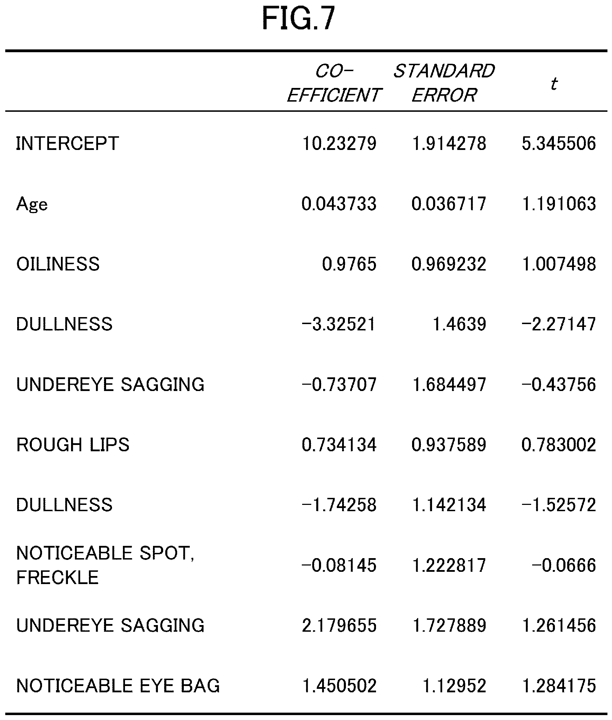

FIG. 7 illustrates coefficients, standard errors, and t-values of "intercept", "age (actual age of subject)", and "questionnaire questions ("oiliness (feeling it, not feeling it)", "dullness (feeling it, not feeling it)", "undereye sagging (feeling it, not feeling it)", "rough lips (feeling it, not feeling it)", "dullness (suffering from it, not suffering from it)", "noticeable spots and freckles (suffering from it, not suffering from it)", "undereye sagging (suffering from it, not suffering from it)", and "noticeable eye bag (suffering from it, not suffering from it)".

Thus, it was found that "actual age and facial skin questionnaire answers" and "the state of blood vessels in the deep part of the face" are correlated. Therefore, the blood vessel state predicting unit 102 can predict, as the state of blood vessels in the deep part of the face (health of blood vessels), a value obtained by weighting the answers (that is, 1 or 0) of each question of the questionnaire by the respective coefficients illustrated in FIG. 7 and summing up the values.

<<Correspondence relationship between the "measurement value of facial skin" and the "state of blood vessels in the deep part of the face">>

A case will be described in which the information relating to the evaluation target person 30 includes "facial skin measurement value (for example, the amount of sagging around the eyes)".

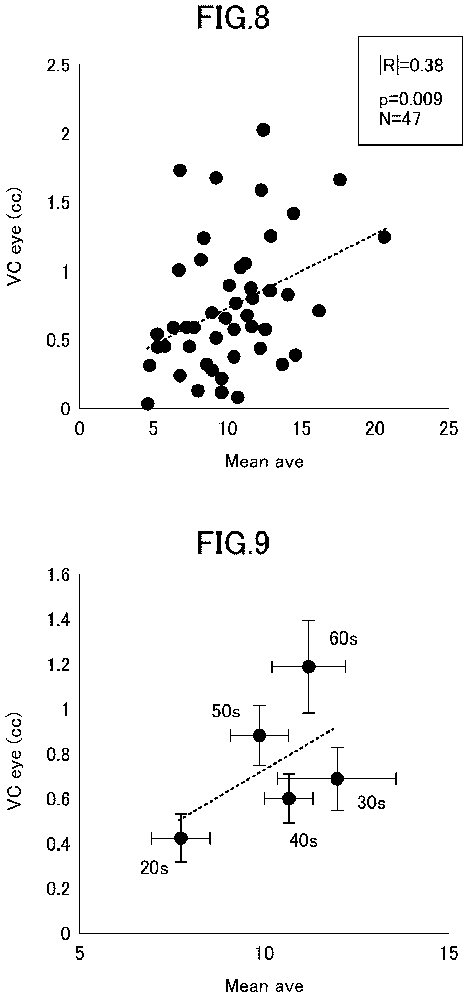

FIG. 8 is a diagram for explaining the relationship between the state of blood vessels (horizontal axis) and the amount of sagging around the eyes (vertical axis) according to an embodiment of the present invention. FIG. 8 is a diagram illustrating the relationship between the state of blood vessels (health of blood vessels) deep in the face of a subject based on the intensity of blood vessel brightness obtained from an MRI image and the amount of sagging around the eyes of the subject. The multiple correlation coefficient was 0.38, the p value was 0.009, and the number of subjects was 47. Thus, the state of blood vessels (health of blood vessels) deep in the face of a subject based on the intensity of blood vessel brightness obtained from an MRI image and the amount of sagging around the eyes of the subject were correlated.

FIG. 9 is a diagram for explaining the relationship between the state of blood vessels (horizontal axis), the amount of sagging around the eyes (vertical axis), and actual age according to an embodiment of the present invention. FIG. 9 illustrates the relationship between the state of blood vessels (health of blood vessels) deep in the face of a subject based on the intensity of blood vessel brightness obtained from an MRI image and the amount of sagging around the eyes of the subject at each age group of actual age. Thus, the state of blood vessels (health of blood vessels) deep in the face of a subject based on the intensity of blood vessel brightness obtained from an MRI image correlated with the amount of sagging around the eyes of the subject, but did not correlate with actual age.

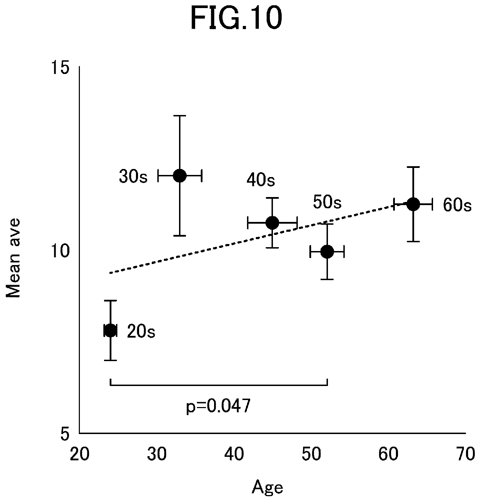

FIG. 10 is a diagram for explaining the relationship between the actual age (horizontal axis) and the state of blood vessels (vertical axis) according to an embodiment of the present invention. FIG. 10 illustrates the relationship between the actual age of a subject and the state of blood vessels (health of blood vessels) deep in the subject's face based on the intensity of blood vessel brightness obtained from an MRI image. The p value for 20s (20's) and 50s (50's) was 0.047 (thus, there was a significant difference between the 20s (20's) and 50s (50's)). However, the actual age of the subject is not correlated with the state of blood vessels (health of blood vessels) deep in the subject's face based on the intensity of blood vessel brightness obtained from an MRI image.

Thus, it was found that the "facial skin measurement value (for example, the amount of sagging around the eyes)" and the "state of blood vessels deep in the face " are more correlated than the actual age and the "state of blood vessels deep in the face". Therefore, the blood vessel state predicting unit 102 can predict the state of blood vessels deep in the face more accurately than using the actual age by using the measurement value (for example, the amount of sagging around the eyes) of the skin of the face.

The skin state predicting unit 103 can predict the measurement value (for example, the amount of sagging around the eyes) of the skin of the face of the evaluation target person 30 from the state of blood vessels deep in the face of the evaluation target person 30 predicted by the blood vessel state predicting unit 102 by using the correlation between the "facial skin measurement value (for example, the amount of sagging around the eyes)" and the "state of blood vessels deep in the face".

<Effects>

According to the present invention, the state of blood vessels deep in the face can be easily identified. For example, the state of blood vessels deep in the face can be identified simply by acquiring the actual age of the evaluation target person and the answer to the questionnaire on the face skin of the evaluation target person. Moreover, for example, the state of blood vessels deep in the face can be identified more accurately than using the actual age simply by acquiring the measurement value of the face skin of the evaluation target person.

Thus, according to the present invention, the state of blood vessels deep in the face that affect the skin can be identified. The conventional method (specifically, a technique based on the structure of blood vessels) can only identify the capillaries affected by the skin, however, according to the method of the present invention, the blood vessels deep in the face that affect the skin can be identified.

<Hardware configuration>

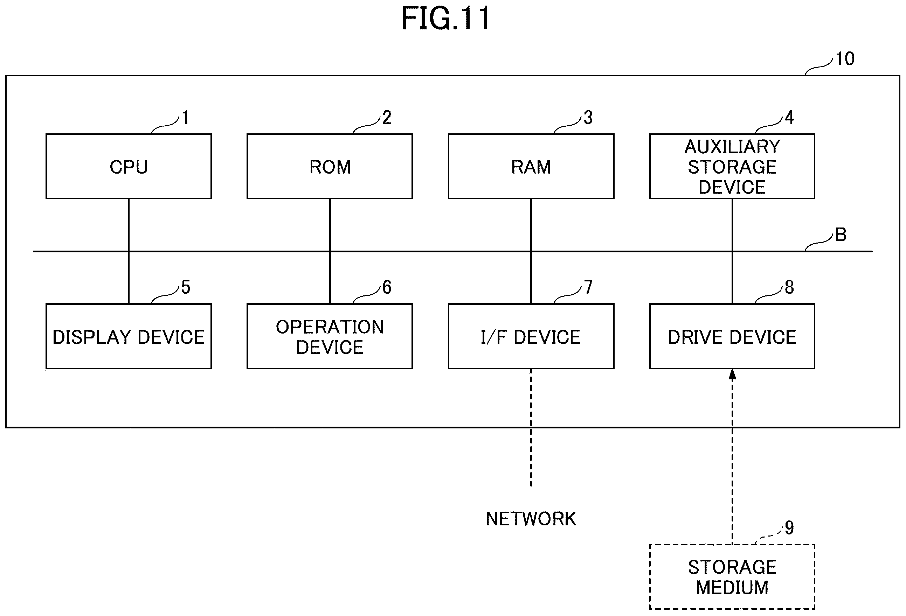

FIG. 11 is a block diagram illustrating an example of the hardware configuration of the evaluation apparatus 10 according to an embodiment of the present invention. The evaluation apparatus 10 includes a central processing unit (CPU) 1, a read only memory (ROM) 2, and a random access memory (RAM) 3. The CPU 1, the ROM 2, and the RAM 3 form a so-called computer. The evaluation apparatus 10 may also include an auxiliary storage device 4, a display device 5, an operation device 6, an interface (I/F) device 7, and a drive device 8. The pieces of hardware of the evaluation apparatus 10 are connected to each other via a bus B.

The CPU 1 is an arithmetic device that executes various programs installed in the auxiliary storage device 4.

The ROM 2 is a nonvolatile memory. The ROM 2 functions as a main storage device that stores various programs and data necessary for the CPU 1 to execute various programs installed in the auxiliary storage device 4. More specifically, the ROM 2 functions as a main storage device that stores boot programs such as BIOS (Basic Input/Output System) and EFI (Extensible Firmware Interface).

The RAM 3 is a volatile memory such as DRAM (Dynamic Random Access Memory) or SRAM (Static Random Access Memory). The RAM 3 functions as a main storage device that provides a work area to be expanded when various programs installed in the auxiliary storage device 4 are executed by CPU 1.

The auxiliary storage device 4 is an auxiliary storage device that stores various programs and information used when various programs are executed.

The display device 5 is a display device that displays the internal state and the like of the evaluation apparatus 10.

The operation device 6 is an input device that the manager of the evaluation apparatus 10 inputs various instructions to the evaluation apparatus 10.

The I/F device 7 is a communication device for connecting to a network and communicating with other devices.

The drive device 8 is a device for setting a storage medium 9. The storage medium 9 herein includes a medium for recording information optically, electrically, or magnetically, such as a CD-ROM, a flexible disk, a magneto-optical disk, and the like. The storage medium 9 may also include a semiconductor memory for electrically recording information, such as an EPROM (Erasable Programmable Read Only Memory), a flash memory, and the like.

The various programs installed in the auxiliary storage device 4 are installed, for example, when the distributed storage medium 9 is set in the drive device 8 and the various programs recorded in the storage medium 9 are read out by the drive device 8. Alternatively, the various programs installed in the auxiliary storage device 4 may be installed by downloading them from the network via the I/F device 7.

Although the embodiments of the present invention have been described above in detail, the present invention is not limited to the specific embodiments described above, and various modifications and changes are possible within the scope of the gist of the invention described in the claims.

This international application claims priority from Japanese Patent Application No. 2022-030316 filed on February 28, 2022, and the entire contents of Japanese Patent Application No. 2022-030316 are hereby incorporated herein by reference.

Reference Signs List

10 evaluation apparatus
20 skin measuring apparatus
30 evaluation target person
101 acquiring unit
102 blood vessel state predicting unit
103 skin state predicting unit
104 presentation unit

**Claims**

1. An evaluation method comprising:

acquiring information relating to an evaluation target person, the information including an actual age of the evaluation target person and an answer to a questionnaire about skin of a face of the evaluation target person; and
predicting a state of a blood vessel in a deep part of the face of the evaluation target person from the information relating to the evaluation target person.

2. An evaluation method comprising:

acquiring information relating to an evaluation target person, the information including a measurement value of skin of a face of the evaluation target person; and
predicting a state of a blood vessel in a deep part of the face of the evaluation target person from the information relating to the evaluation target person.

3. The evaluation method according to claim 1, further comprising:
predicting a measurement value of the skin of the face of the evaluation target person from the state of the blood vessel at the deep part of the face of the evaluation target person.

4. The evaluation method according to claim 2 or 3, wherein the measurement value of the skin of the face of the evaluation target person is an amount of sagging around eyes of the evaluation target person.

5. The evaluation method according to any one of claims 1 to 4, further comprising:
presenting beauty information according to the state of the blood vessel in the deep part of the face of the evaluation target person.

6. The evaluation method according to any one of claims 1 to 5, wherein the blood vessel in the deep part of the face is an artery.

7. The evaluation method according to any one of claims 1 to 6, wherein the state of the blood vessel in the deep part of the face is a blood vessel state calculated from a blood flow velocity in the blood vessel.

8. The evaluation method according to any one of claims 1 to 6, wherein the state of the blood vessel in the deep part of the face is a blood vessel state calculated from a flow rate of blood in the blood vessel and a radius of the blood vessel.

9. The evaluation method according to any one of claims 1 to 6, wherein the state of the blood vessel in the deep part of the face is a blood vessel state calculated from a signal intensity in the blood vessel in an MRI(Magnetic Resonance Imaging) image.

10. The evaluation method according to any one of claims 1 to 6, wherein the state of the blood vessel in the deep part of the face is a blood vessel state calculated from a signal intensity in the blood vessel in an MRA-PC (Magnetic Resonance Angiography - Phase Contrast) image in an MRI(Magnetic Resonance Imaging) image.

11. An evaluation apparatus comprising:

an acquiring unit configured to acquire information relating to an evaluation target person, the information including an actual age of the evaluation target person and an answer to a questionnaire about skin of a face of the evaluation target person; and
a predicting unit configured to predict a state of a blood vessel in a deep part of the face of the evaluation target person from the information relating to the evaluation target person.

12. An evaluation apparatus comprising:

an acquiring unit configured to acquire information relating to an evaluation target person, the information including a measurement value of skin of a face of the evaluation target person; and
a predicting unit configured to predict a state of a blood vessel in a deep part of the face of the evaluation target person from the information relating to the evaluation target person.

13. A program that causes an evaluation apparatus to function as:

an acquiring unit configured to acquire information relating to an evaluation target person, the information including an actual age of the evaluation target person and an answer to a questionnaire about skin of a face of the evaluation target person; and
a predicting unit configured to predict a state of a blood vessel in a deep part of the face of the evaluation target person from the information relating to the evaluation target person.

14. A program that causes an evaluation apparatus to function as:

an acquiring unit configured to acquire information relating to an evaluation target person, the information including a measurement value of skin of a face of the evaluation target person; and
a predicting unit configured to predict a state of a blood vessel in a deep part of the face of the evaluation target person from the information relating to the evaluation target person.

# FIG.1

30

EVALUATION
TARGET PERSON

10

EVALUATION
APPARATUS

20

SKIN MEASURING
APPARATUS

# FIG.2

EVALUATION APPARATUS — 10

INFORMATION RELATING TO EVALUATION TARGET PERSON → ACQUIRING UNIT (101)

→ (INFORMATION RELATING TO EVALUATION TARGET PERSON) → BLOOD VESSEL STATE PREDICTING UNIT (102)

→ (STATE OF BLOOD VESSEL IN DEEP PART OF FACE) → PRESENTATION UNIT (104) → BEAUTY INFORMATION

STATE OF BLOOD VESSEL IN DEEP PART OF FACE → SKIN STATE PREDICTING UNIT (103)

MEASUREMENT VALUE OF SKIN OF FACE → PRESENTATION UNIT (104)

EP 4 487 760 A1

# FIG.3

```
   ┌─────────┐
   │  START  │
   └─────────┘
        │
        ▼                                          ⟋S101
┌───────────────────────────────────────────────────┐
│   ACQUIRE ACTUAL AGE OF EVALUATION TARGET AND      │
│            QUESTIONNAIRE ANSWERS                    │
│  ABOUT SKIN OF FACE OF EVALUATION TARGET PERSON    │
└───────────────────────────────────────────────────┘
        │
        ▼                                          ⟋S102
┌───────────────────────────────────────────────────┐
│          PREDICT STATE OF BLOOD VESSEL             │
│  IN DEEP PART OF FACE OF EVALUATION TARGET PERSON  │
└───────────────────────────────────────────────────┘
        │
        ▼                                          ⟋S103
┌───────────────────────────────────────────────────┐
│          PREDICT MEASUREMENT VALUE OF              │
│    SKIN OF FACE OF EVALUATION TARGET PERSON        │
└───────────────────────────────────────────────────┘
        │
        ▼                                          ⟋S104
┌───────────────────────────────────────────────────┐
│          PRESENT BEAUTY INFORMATION                │
└───────────────────────────────────────────────────┘
        │
        ▼
   ┌─────────┐
   │   END   │
   └─────────┘
```

# FIG.4

START

S201

ACQUIRE MEASUREMENT VALUE OF
SKIN OF FACE OF EVALUATION TARGET PERSON

S202

PREDICT STATE OF BLOOD VESSEL
IN DEEP PART OF FACE OF EVALUATION TARGET PERSON

S203

PRESENT BEAUTY INFORMATION

END

# FIG.5

| No | Ave | Age | OILINESS | DULLNESS | UNDEREYE SAGGING | ROUGH LIPS | DULLNESS | NOTICEABLE SPOT, FRECKLE | UNDEREYE SAGGING | NOTICE-ABLE EYE BAG |
|---|---|---|---|---|---|---|---|---|---|---|
| 1 | 4.66 | 23 | 1 | 1 | 0 | 1 | 1 | 1 | 0 | 0 |
| 2 | 8.015 | 23 | 1 | 0 | 0 | 1 | 0 | 0 | 0 | 0 |
| 3 | 10.87 | 24 | 0 | 1 | 1 | 1 | 1 | 0 | 1 | 0 |
| 4 | 10.725 | 24 | 1 | 1 | 0 | 1 | 1 | 1 | 0 | 0 |
| 5 | 8.62 | 25 | 0 | 1 | 0 | 0 | 1 | 1 | 0 | 0 |
| 6 | 5.345 | 23 | 0 | 1 | 0 | 0 | 0 | 0 | 0 | 1 |
| 7 | 4.78 | 25 | 0 | 1 | 0 | 1 | 1 | 1 | 0 | 0 |
| 8 | 9.245 | 24 | 1 | 1 | 0 | 1 | 1 | 1 | 1 | 0 |
| 9 | 10.15 | 24 | 1 | 1 | 1 | 0 | 1 | 1 | 1 | 0 |
| 10 | 12.6 | 33 | 0 | 0 | 0 | 0 | 0 | 0 | 0 | 0 |
| 11 | 5.77 | 30 | 0 | 1 | 0 | 0 | 1 | 1 | 0 | 0 |
| 12 | 16.25 | 30 | 1 | 0 | 1 | 1 | 0 | 1 | 1 | 1 |
| 13 | 20.7 | 37 | 1 | 0 | 0 | 1 | 0 | 0 | 0 | 0 |
| 14 | 6.405 | 32 | 1 | 1 | 0 | 1 | 1 | 1 | 0 | 0 |
| 15 | 17.65 | 31 | 1 | 0 | 0 | 0 | 0 | 0 | 0 | 0 |
| 16 | 7.285 | 32 | 0 | 1 | 0 | 1 | 0 | 0 | 0 | 0 |
| 17 | 9.65 | 35 | 1 | 1 | 0 | 1 | 0 | 0 | 0 | 0 |
| 18 | 13.7 | 38 | 1 | 0 | 1 | 1 | 1 | 0 | 1 | 1 |
| 19 | 9.9 | 32 | 1 | 1 | 0 | 1 | 0 | 1 | 0 | 0 |
| 20 | 14.6 | 40 | 1 | 1 | 1 | 1 | 1 | 1 | 1 | 1 |
| 21 | 12.25 | 44 | 0 | 1 | 1 | 1 | 1 | 0 | 1 | 1 |
| 22 | 11.595 | 48 | 0 | 1 | 1 | 0 | 0 | 1 | 0 | 0 |
| 23 | 9.725 | 41 | 1 | 1 | 0 | 1 | 0 | 1 | 0 | 0 |
| 24 | 8.255 | 44 | 1 | 1 | 1 | 0 | 0 | 1 | 1 | 0 |
| 25 | 9.02 | 47 | 1 | 1 | 0 | 0 | 1 | 1 | 0 | 0 |
| 26 | 10.5 | 45 | 0 | 1 | 1 | 1 | 1 | 1 | 1 | 0 |
| 27 | 11.255 | 48 | 1 | 1 | 1 | 1 | 1 | 1 | 1 | 0 |
| 28 | 9.055 | 49 | 1 | 1 | 1 | 1 | 1 | 1 | 1 | 1 |
| 29 | 13 | 50 | 1 | 1 | 1 | 1 | 1 | 1 | 1 | 1 |
| 30 | 11.775 | 53 | 1 | 1 | 1 | 1 | 1 | 1 | 1 | 1 |
| 31 | 12.95 | 54 | 1 | 1 | 1 | 1 | 1 | 1 | 1 | 1 |
| 32 | 9.3 | 55 | 0 | 0 | 1 | 1 | 0 | 1 | 0 | 0 |
| 33 | 7.815 | 50 | 0 | 1 | 1 | 0 | 1 | 1 | 1 | 1 |
| 34 | 6.805 | 56 | 0 | 1 | 0 | 0 | 1 | 0 | 0 | 0 |
| 35 | 6.795 | 53 | 1 | 1 | 1 | 0 | 1 | 1 | 1 | 0 |
| 36 | 10.525 | 52 | 0 | 0 | 1 | 0 | 0 | 0 | 0 | 0 |
| 37 | 11.715 | 50 | 0 | 1 | 1 | 1 | 1 | 1 | 1 | 1 |
| 38 | 8.45 | 50 | 1 | 1 | 1 | 0 | 1 | 1 | 1 | 0 |
| 39 | 7.415 | 63 | 0 | 1 | 1 | 0 | 1 | 1 | 0 | 0 |
| 40 | 14.125 | 62 | 0 | 1 | 0 | 0 | 0 | 1 | 0 | 0 |
| 41 | 12.45 | 61 | 0 | 1 | 1 | 1 | 0 | 1 | 1 | 0 |
| 42 | 10.625 | 65 | 0 | 1 | 1 | 0 | 1 | 1 | 1 | 1 |
| 43 | 11.385 | 69 | 1 | 0 | 0 | 0 | 0 | 0 | 0 | 0 |
| 44 | 12.4 | 63 | 0 | 1 | 1 | 1 | 1 | 1 | 1 | 1 |
| 45 | 14.45 | 64 | 1 | 1 | 1 | 1 | 1 | 1 | 1 | 1 |
| 46 | 6.82 | 61 | 0 | 1 | 1 | 1 | 1 | 1 | 1 | 0 |

# FIG.6

## *REGRESSION STATISTICS*

| | |
|---|---|
| MULTIPLE CORRELATION R | 0.661344 |
| MULTIPLE DETERMINATION R2 | 0.437376 |
| CORRECTION R2 | 0.29672 |
| STANDARD ERROR | 2.809063 |
| NUMBER OF OBSERVATIONS | 46 |

# FIG.7

|  | CO-EFFICIENT | STANDARD ERROR | t |
|---|---|---|---|
| INTERCEPT | 10.23279 | 1.914278 | 5.345506 |
| Age | 0.043733 | 0.036717 | 1.191063 |
| OILINESS | 0.9765 | 0.969232 | 1.007498 |
| DULLNESS | −3.32521 | 1.4639 | −2.27147 |
| UNDEREYE SAGGING | −0.73707 | 1.684497 | −0.43756 |
| ROUGH LIPS | 0.734134 | 0.937589 | 0.783002 |
| DULLNESS | −1.74258 | 1.142134 | −1.52572 |
| NOTICEABLE SPOT, FRECKLE | −0.08145 | 1.222817 | −0.0666 |
| UNDEREYE SAGGING | 2.179655 | 1.727889 | 1.261456 |
| NOTICEABLE EYE BAG | 1.450502 | 1.12952 | 1.284175 |

# FIG.8

# FIG.9

# FIG.10

# FIG.11

EP 4 487 760 A1

## INTERNATIONAL SEARCH REPORT

| International application No. |
|---|
| **PCT/JP2023/004952** |

### A. CLASSIFICATION OF SUBJECT MATTER

***A61B 5/00***(2006.01)i; ***A61B 5/055***(2006.01)i
FI:   A61B5/00 M; A61B5/055 380

According to International Patent Classification (IPC) or to both national classification and IPC

### B. FIELDS SEARCHED

Minimum documentation searched (classification system followed by classification symbols)

   A61B5/00; A61B5/055

Documentation searched other than minimum documentation to the extent that such documents are included in the fields searched

   Published examined utility model applications of Japan 1922-1996
   Published unexamined utility model applications of Japan 1971-2023
   Registered utility model specifications of Japan 1996-2023
   Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | 資生堂、毛細血管が肌の断量を生み出すメカニズムを解明, 株式会社資生堂Ｐｒｅｓｓ Ｒｅｌｅａｓｅ [online], 株式会社資生堂, 25 May 2020 [retrieved on: 04 April 2023], Internet: <URL: https://corp.shiseido.com/jp/newsimg/2911_l1q96_jp.pdf>, non-official translation (Shiseido revealed the mechanism in which capillary vessels produce skin elasticity. Shiseido Co., Ltd. Press Release) pp. 1-2 | 1-3, 5-8, 11-14 |
| Y | | 9-10 |
| A | | 4 |
| Y | US 2021/0282703 A1 (SAMSUNG ELECTRONICS CO., LTD.) 16 September 2021 (2021-09-16) paragraph [0170] | 9-10 |
| Y | US 2021/0082545 A1 (ENLITIC, INC.) 18 March 2021 (2021-03-18) paragraph [0059] | 9-10 |
| Y | WO 2020/170160 A1 (AUGMENTED ANATOMY BVBA) 27 August 2020 (2020-08-27) p. 14, line 23 | 9-10 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 April 2023** | **18 April 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2023/004952** |

**Box No. III      Observations where unity of invention is lacking (Continuation of item 3 of first sheet)**

This International Searching Authority found multiple inventions in this international application, as follows:

(Invention 1) Claims 1, 11, and 13
An evaluation method including "acquiring information related to a subject of evaluation, including the actual age of the subject of evaluation, and answers to a questionnaire about the skin of the face of the subject of evaluation".

(Invention 2) Claims 2, 12, and 14
An evaluation method including "acquiring information related to the subject of evaluation, including measurement values of the skin of the face of the subject".

Claim 3, claim 4 selectively citing claim 3, and claims 5-10 selectively citing claim 1 or 3 are included in invention 1. Claim 4 selectively citing claim 2, and claims 5-10 selectively citing claim 2 or 4 are included in invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2023/004952**

| Patent document cited in search report | | | Publication date (day/month/year) | Patent family member(s) | | | Publication date (day/month/year) |
|---|---|---|---|---|---|---|---|
| US | 2021/0282703 | A1 | 16 September 2021 | CN | 109715050 | A | |
| | | | | KR | 10-2019-0068500 | A | |
| US | 2021/0082545 | A1 | 18 March 2021 | WO | 2022/036351 | A1 | |
| WO | 2020/170160 | A1 | 27 August 2020 | BE | 1027062 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008188428 A

- JP 2022030316 A